# EUROPÄISCHE PATENTSCHRIFT

(11) **EP 0 533 058 B1**
(45) Veröffentlichungstag und Bekanntmachung des Hinweises auf die Patenterteilung: **20.08.2003**
(21) Anmeldenummer: 92115500.8
(22) Anmeldetag: 10.09.1992
(51) Int. Cl.: C07D 235/08, A61K 31/415, C07D 409/06, C07D 471/04, C07D 487/04, C07D 519/00

(54) **Imidazo-annellierte Iso- und Heterocyclen, Antagonisten als Angiotensin II**
Imidazo-fused iso- and heterocycles as angiotensin II antagonists
Iso- et hétérocycles imidazo-condensés comme antagonistes d'angiotensine II

(30) Priorität: 14.09.1991 DE 4130659; 20.09.1991 DE 4131325
(43) Veröffentlichungstag der Anmeldung: 24.03.1993
(73) Patentinhaber: HOECHST AKTIENGESELLSCHAFT, 65926 Frankfurt am Main (DE)
(72) Erfinder: Heitsch, Holger, Dr., W-6238 Hofheim (Ts.) (DE); Wagner, Adalbert, Dr., W-6234 Hattersheim/M. (DE); Kleemann, Heinz-Werner, Dr., W-6380 Bad Homburg (DE); Gerhards, Hermann, Dr., W-6238 Hofheim (Ts.) (DE); Schölkens, Bernward, Prof. Dr., W-6233 Kelkheim (DE)

(56) Entgegenhaltungen:
- EP-A- 0 151 094
- EP-A- 0 392 317
- EP-A- 0 399 731
- EP-A- 0 399 732
- EP-A- 0 400 835
- EP-A- 0 400 974
- EP-A- 0 425 921
- EP-A- 0 430 709
- EP-A- 0 461 039
- EP-A- 0 470 543

## Beschreibung

Aus EP-A 399 731, EP-A 399 732, EP-A 400 835 und EP-A 434 038 sind imidazoanellierte, aromatische Verbindungen als Antagonisten von Angiotensin-II-Rezeptoren bekannt.

In EP-A 461 039 werden Benzimidazole, in EP-A 470 543 heterocyclische Imidazole beschrieben, die eine Biphenylgruppe am Stickstoff des Imidazol tragen.
Jedoch beschreibt keine der obengenannten Literaturstellen Imidazopyridinderivate, die eine Biphenylgruppe am Stickstoff des Imidazol und gleichzeitig an der Biphenylgruppe einen Sulfonylharnstoff- oder Sulfonylurethanrest tragen.

Es wurden nun Imidazopyridinderivate gefunden, die überraschenderweise hochwirksame Antagonisten von Angiotensin-ll-Rezeptoren sowohl in vitro als auch in vivo sind.

Die Erfindung betrifft Verbindungen der Formel (I) 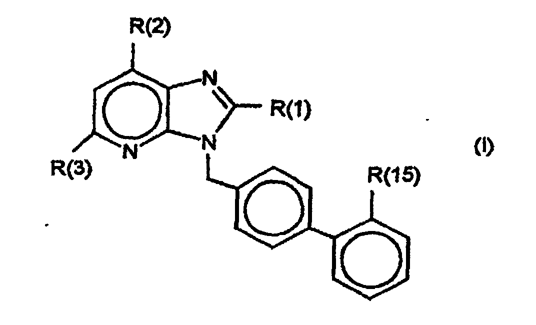 worin bedeuten
a)
   - R(1): Ethyl;
   - R(2): Methyl;
   - R(3): Wasserstoff;
   - R(15): 1. - SO₂-NH-CO-O-R(17) mit
      R(17) gleich Methyl, Ethyl, Propyl, Vinyl oder Benzyl;
   2. - SO₂-NH-CO-NH-R(17) mit
      R(17) gleich Methyl, Ethyl, Propyl, Benzyl, Cyclohexyl, Cyclopropylmethyl oder Allyl;
   3. - SO₂-NH-CO-N(CH₃)-R(17) mit
      R(17) gleich Methyl oder Propyl; oder
   4. -SO₂-NH-CO-N ;
b)
   - R(1): Ethyl;
   - R(2): Methyl;
   - R(3): Methyl;
   - R(15): 1. - SO₂-NH-CO-O-R(17) mit
      R(17) gleich Methyl, Ethyl, Propyl, Cyclopropylmethyl, Benzyl oder Cyclohexylmethyl;
   2. - SO₂-NH-CO-NH-R(17) mit
      R(17) gleich Methyl, Ethyl, Propyl, Allyl, Cyclopropylmethyl, Cyclohexylmethyl oder Phenylethyl;
   3. SO₂NH-CO-N(Allyl)₂;
   4. SO₂N-(CO-O-Allyl)₂; oder
   5. SO₂N-(CO-O-Benzyl)₂;
c)
   - R(1): n-Butyl;
   - R(2): Wasserstoff;
   - R(3): Wasserstoff;
   - R(15): 1. -SO₂-NH-CO-O-R(17) mit
      R(17) gleich Ethyl oder Benzyl; oder
   2. -SO₂-NH-CO-NH-R(17) mit
      R(17) gleich n-Propyl; iso-Propyl oder Allyl;
sowie deren physiologisch verträglichen Salze.

Unter physiologisch verträglichen Salzen von Verbindungen der Formel I versteht man sowohl deren organische als auch anorganische Salze, wie sie in Remington's Pharmaceutical Sciences, 17. Auflage, Seite 1418 (1985) beschrieben sind. Aufgrund von physikalischer und chemischer Stabilität und Löslichkeit sind für saure Gruppen unter anderem Natrium-, Kalium-, Calcium- und Ammoniumsalze bevorzugt, für basische Gruppen unter anderen Salze mit Salzsäure, Schwefelsäure, Phosphorsäure, Carbonsäuren oder Sulfonsäuren, wie Essigsäure, Zitronensäure, Benzoesäure, Maleinsäure, Fumarsäure, Weinsäure, p-Toluolsulfonsäure.

Die Erfindung betrifft auch ein Verfahren zur Herstellung von Verbindungen der Formel I, das dadurch gekennzeichnet ist, daß man Verbindungen der Formel II 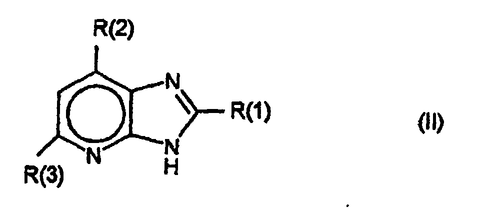 worin R(1), R(2) und R(3) wie oben definiert sind, alkyliert mit Verbindungen der Formel III 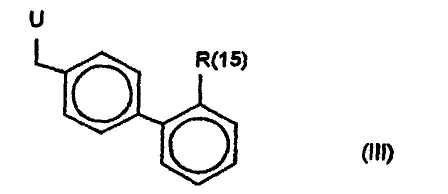 worin R(15) wie oben definiert ist und U für eine Fluchtgruppe steht, gegebenenfalls eingeführte Schutzgruppen wieder abgespaltet und die erhaltenen Verbindungen der Formel I gegebenenfalls in ihre physiologisch verträglichen Salze überführt werden.

Geeignete Fluchtgruppen U sind vorzugsweise nucleofuge Gruppen (vgl. Angew. Chem. 72 (1960)) wie Halogen, o-Toluolsulfonat, Mesylat oder Triflat.

Verfahren zur Herstellung der Vorstufen der Formel II sind u.a. bekannt aus US 4 880 804, DE 3 911 603, EP-A-399 731, EP-A-399 732, EP-A-400 835, EP-A-400 974, EP-A-415 886, EP-A-420 237, EP-A-425 921 und EP-A-434 038.

Zur Alkylierung der Verbindungen der Formel II sind z.B. entsprechende Benzylhalogenide, -tosylate, -mesylate oder -triflate oder entsprechende Alkylhalogenide, -tosylate, -mesylate oder -triffate geeignet.

Die Darstellung dieser Verbindungen erfolgt in an sich bekannter Weise, beispielsweise durch Halogenierung der entsprechenden Methylvorstufen. Hierzu wird vorzugsweise N-Bromsuccinimid eingesetzt, siehe z.B. J. Org. Chem. 44, 4733 (1979) und Helv. Chim. Acta 62, 2661 (1979).

Die Synthese der Benzimidazol-, Benzthiophen-, Imidazo-pyridin- und Imidazopyrimidin-Derivate mit CH₃-Gruppe am Kern erfolgte unter anderem nach R.P. Dickson et al. in J. Med. Chem. 29, 1637 (1986), E. Abignente et al. in J. Heterocyclic Chem. 26, 1875 (1989), A. Koubsack et al. in J. Org. Chem. 41, 3399 (1976) und nach F. Santer et al. in Mh. Chem. 99, 715 (1968).

Die Biphenylderivate können z.B. ausgehend von Arylboronsäurederivaten durch Kopplung mit substituierten Arylhalogeniden mit Übergangsmetallkatalysatoren, insbesondere Palladium synthetisiert werden. Entsprechende Reaktionen werden von R.B. Miller et al. (Organometallics 1984, 3, 1261) oder von A. Zuzuki et al. (Synthetic Commun. 11 (7), 513 (1981)) beschrieben.

Die Sulfonylurethan-Derivate der Formel (I) können aus entsprechenden Sulfonamiden der Formel (I) durch Umsetzung mit Chlorkohlensäureestern oder durch Umsatz mit Dimethyldicarbonat und Basen wie z.B. Kaliumcarbonat in inerten Lösungsmitteln bei Temperaturen bis zum Siedepunkt des entsprechenden Lösungsmittels erhalten werden.

Die Sulfonylharnstoff-Derivate der Formel (I) sind wahlweise entweder aus den entsprechenden Sulfonamiden der Formel (I) durch Umsetzung mit Isocyanaten oder mit 2,2,2-Trichloracetamid-Derivaten eines geeigneten Amins in inerten, hochsiedenden Lösungsmitteln wie z.B. DMSO oder aus Sulfonylurethanen der Formel (I) durch Einwirkung des entsprechenden Amins in einem inerten, hochsiedenden Lösungsmittel wie z.B. Toluol bei Temperaturen bis zum Siedepunkt des jeweiligen Lösungsmittels darstellbar.

Der Sulfonamid-Rest kann, falls erforderlich, ausgehend von einer Aminogruppe mittels Meerwein-Umlagerung hergestellt werden. Hierfür wird das Hydrochlorid des Amins zuerst diazotiert und dann in Gegenwart eines Kupferkatalysators mit Schwefeldioxid in Eisessig umgesetzt Nachfolgende Einwirkung von Ammoniak führt zur Sulfonamidogruppe.

Die Alkylierung erfolgt nach prinzipiell bekannten Verfahren in analoger Weise.

Die Imidazo-anellierten Derivate der Formel II werden z.B. in Gegenwart einer Base metalliert. Bevorzugte Basen sind Metallhydride der Formel MH wie Lithium-, Natrium- oder Kaliumhydrid in beispielsweise DMF oder DMSO als Lösungsmittel oder Metallalkoxide der Formel MOR, wobei R für Methyl, Ethyl, t-Butyl steht, und die Reaktion in dem entsprechenden Alkohol, DMF oder DMSO durchgeführt wird. Die so gebildeten Salze der Imidazo-Derivate werden in einem aprotischen Lösungsmittel wie DMF oder DMSO gelöst und mit einer geeigneten Menge Alkylierungsreagenz versetzt.

Eine alternative Möglichkeit zur Deprotonierung der Imidazol-Derivate stellt z.B. die Umsetzung mit Kaliumcarbonat in DMF oder DMSO dar.

Die Umsetzungen werden bei Temperaturen unterhalb Zimmertemperatur bis zum Siedepunkt des Reaktionsgemisches, vorzugsweise zwischen +20°C und dem Siedepunkt des Reaktionsgemisches während etwa 1 bis 10 Stunden durchgeführt.

Die erfindungsgemäßen Verbindungen der Formel I haben antagonistische Wirkung auf Angiotensin-II-Rezeptoren und können deshalb zur Behandlung der Angiotensin-II-abhängigen Hypertension verwendet werden. Anwendungsmöglichkeiten bestehen weiterin bei Herzinsuffizienz, Kardioprotektion, Myocardinfarkt, Herz-Hyperthrophie, Arteriosklerose, Nephropathie, Nierenversagen sowie cardiovasculären Erkrankungen des Gehirn wie transitorischen ischämischen Attacken und Gehirnschlag.

Renin ist ein proteolytisches Enzym aus der Klasse der Aspartyl-proteasen, welches als Folge verschiedener Stimuli (Volumendepletion,, Natriummangel, β-Rezeptorenstimulation) vor den juxtaglomerulären Zellen der Niere in den Blutkreislauf sezerniert wird. Dort spaltet es von dem aus der Leber ausgeschiedenen Angiotensinogen das Dexapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt.

Postrezeptor-Wirkungen sind unter anderen Stimulation des Phosphoinositol-Umsatzes (Ca²⁺-Freisetzung), Aktivierung der Proteinkinse C und Facilitation von AMP-abhängigen Hormon-Rezeptoren.

Die Affinität der Verbindungen der Formel I zum Angiotensin-II-Rezeptor kann durch Messung der ¹²⁵I-Angiotensin-II- oder ³H-Angiotensin-II-Verdrängung von Rezeptoren an Zona glomerulosa Membranen von Rindemebennieren bestimmt werden. Dazu werden die präparierten Membranen in Puffer bei pH 7,4 suspendiert. Um die Degradierung des Radioliganden während der Inkubierung zu verhindern, wird Aprotinin, ein Peptidase-Inhibitor, zugesetzt Zusätzlich werden ca. 14000 cpm eines Tracers mit spezifischer Aktivität von 74 TBg/mmol (käuflich bei Amersham Buchler) und eine Menge Rezeptorprotein, die 50 % des Tracers bindet, verwendet. Die Reaktion wird durch Zugabe von 50 µl Membransuspension zu einer Mischung von 100 µl Puffer + Aprotinin; 50 µl Puffer mit oder ohne Angiotensin-II oder Rezeptorantagonist und 50 µl Tracer gestartet. Nach einer Inkubationszeit von 60 Minuten bei 25°C werden gebundener und freie Radioligand durch einen Filtrationsassay mit Whatmann^{(R)} GFIC Filtern auf einem Skatron^{(R)}-Zellsammler getrennt.

Unspezifische Bindungen werden durch Behandlung der Filter mit 0,3 % Polyethylenimin pH = 10 verhindert (Sigma, Nr. 3143). Durch Messung der Radioaktivität in einem Gamma-Szintillationszähler wird die Stärke der Verdrängung des Radioliganden vom Rezeptor bestimmt. Die IC₅₀-Werte, welche die Konzentration des Inhibitors bedeutet, um 50 % des Uganden zu verdrängen, werden nach Chem. et at. J. Theor. Biol. 59, 253 (1970) bestimmt. Sie liegen für die Verbindungen der Formel (I) im Bereich von 1x10⁻⁴ - 1x10⁻⁹ M.

Alternativ kann die Affinität der Verbindungen der Formel I zum Angiotensin-II-Rezeptor durch Messung der ¹²⁵I-Angiotensin-II- oder ³H-Angiotensin-II-Verdrängung von Rezeptorpräparationen aus verschiedenen Organen (Leber, Lunge, Nebenniere, Hirn etc.) bestimmt werden.

Dazu werden die präparierten Membranen in einem Inkubationspuffer (20 mM Tris, pH 7.4, enthaltend 135 mM NaCl, 10 mM KCl, 10 mM MgCl₂, 5 mM Glucose, 0.2 % Rinderserumalbumin sowie die Proteaseinhibitoren PMSF 0,3 mM und Bacitracin 0,1 mM) suspendiert und zusammen mit dem radioaktiv markierten Angiotensin-II und verschiedenen Konzentrationen der zu testenden Verbindungen für 90 min bei 25°C inkubiert. Anschließend werden gebundener und freier Radioligand durch Filtration über Microglasfaserfilter (GF51, Schleicher & Schüll) auf einem Zellsammler (SKATRON) getrennt.

Durch Messung der Rezeptor-gebundenen Radioaktivität auf den Filtern mittels eines Beta- oder Gamma-Spektrometers wird der Grad derVerdrängung des Radioliganden vom Rezeptor durch die Testverbindungen bestimmt. Die Stärke der Verdrängung des Radioliganden vom Rezeptor durch die Testverbindungen wird mit der IC₅₀ angegeben, d.i. die Konzentration des Inhibitors, die 50 % des gebundenen Radioliganden vom Rezeptor verdrängt Die Berechnung der IC₅₀-Werte erfolgt mittels einer PC-Software (LIGAND, G.A. McPherson 1985, Elsevier-BIOSOFT, 68 Hills Road, Cambridge CB 21LA, UK.). Die für Verbindungen der Formel (I) gemessenen IC₅₀ Werte liegen im Bereich von 1 x 10⁻⁵ bis 1 x 10⁻¹¹ M.

Zur Bestimmung der antagonistischen Wirkung der Verbindungen der Formel (I) in vivo kann ihr inhibierender Effekt auf den Angiotensin-ll-induzierten Blutdruckanstieg an entmarkten Sprague-Dawley-Ratten (Möllegard, Dänemark) gemessen werden. Der Blutdruck wird an der Aorta carotis gemessen. Die i.v.-Applikation erfolgt in die Penis-Vene. Nach Präparation des Tieres und 20-minütiger Wartezeit zur Stabilisierung der hämodynamischen Parameter werden 3 aufeinanderfolgende Injektionen von 10, 30 und 100 ng Angiotensin-II in 0,1 ml wäßriger Lösung in 5-Minuten-Intervallen verabreicht. Die Verbindungen der Formel (I) werden in destilliertem Wasser,eventuell unter 10 %igem Ethanol- und/oder Basen-(pH < 10) oder Säuren-(pH > 3) Zusatz, gelöst und in Dosen von 1-300 µg/kg intravenös bzw. 5-1000 µg/kg intraduodenal appliziert.
Bei intraduodenaler Gabe erfolgt die Angiotensin-II-Injektion nach 20, 40 und 60 Minuten, während bei intravenöser Gabe die pressor response sequence in 10-Minuten-Intervallen erfolgt.
Die Verbindungen der Formel (I) sind insbesondere im Bereich von 1-300 µg/kg intravenös bzw. 5-300 µg/kg intraduodenal wirksam.

Die Erfindung bezieht sich ebenso auf pharmazeutische Zusammensetzung bestehend aus einer Verbindung der Formel I und anderen Wirkstoffen, wie z.B. Diuretika oder nichtstereoidaler antiflammatorischen Wirkstoffen. Die Verbindungen der Formel I können auch als Diagnostika für das Renin-Angiotensin-System verwendet werden.

Pharmazeutische Präparate enthalten eine wirksame Menge des Wirkstoffs der Formel I und eventuell andere Wirkstoffe zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methode in geeignete Darreichungsformen gebracht; wie Tabletten, Dragees, Steckkapseln, wäßrige alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittlern, Emulgatoren oder weiteren Hilfsstoffen in Lösungen, Suspensionen oder Emulsionen gebracht. Als Lösungsmittel kommen z.B. in Frage: Wasser, physiologische Kochsalzlösung oder Alkohole, z.B. Ethanol, Propandiol oder Glycerin, daneben auch Zuckerlösungen wie Glucose oder Mannitlösungen oder auch eine Mischung aus den verschiedenen genannten Lösungsmitteln.

Nach dem oben angeführten Verfahren wurden beispielsweise für die Verbindungen der Beispiele 4, 10, 14 und 34 folgende IC₅₀-Werte bestimmt:

| Beispiel | IC₅₀ [nM] |
|---|---|
| 4 | 0,8 |
| 10 | 1,1 |
| 14 | 0,48 |
| 34 | 1,8 |

### Liste der Abkürzungen:

- DCI: Desorption-Chemical Ionisation
- DMF: N,N-Dimethylformamid
- EE: Ethylacetat
- FAB: Fast Atom Bombardment
- h: Stunde(n)
- Hep: n-Heptan
- Min: Minute(n)
- NBS: N-Bromsuccinimid
- RT: Raumtemperatur

### Referenzbeispiel 1

### 2-n-Butyl-1-[(2-carboxy-3-chlor-benzo[b]thiophen-6-yl)-methyl]-1H-benzimidazol-7-carbonsäure

### a) 2-Carboxy-6-nitro-benzoesäureamid

30 g (0,155 mol) 3-Nitrophthalsäureanhydrid werden portionsweise in 180 ml konz. Ammoniak-Lösung eingetragen und die restulierende Lösung unter Rühren 45 Min. bei 100°C erhitzt. Es wird am Rotationsverdampfer eingedampft, 2 x mit Toluol codestilliert und der Rückstand im Hochvakuum getrocknet. Es wird mit EE verrührt, der beige Niederschlag abgesaugt und im Vakuum über P₂O₅ getrocknet. Es werden 31,8 g der Titelverbindung gewonnen.
Schmp.: 188°C
R_{f} (SiO₂, CH₂Cl₂/MeOH 1:1) = 0,3
MS (DCl): 211 (M+H)

### b) 2-Amino-3-nitro-benzoesäure

31 g (0,147 mol) der Verbindung aus Referenzbeispiel 1a) werden in 50 ml 4N Natronlauge und 100 ml Wasser gelöst, 150 ml Natriumhypochlorid-Lösung (Überschuß gegen Kl-Stärkepapier) zugefügt und die erhaltene Lösung 60 Min bei 100°C erhitzt. Nach Ende der Reaktion wird abgekühlt, mit 250 ml ges. Na₂CO₃-Lösung und 400 ml ges. KH₂PO₄-Lösung versetzt, der pH der Lösung mit 4n HCl/konz.
HCI auf 3 eingestellt und das Produkt 3x mit je 500 ml EE extrahiert. Nach Trocknung über MgSO₄, Einengung und Verrühren mit Diisopropylether werden 18 g der Titelverbindung gewonnen.
Schmp.: 188-194°C
R_{f} (SiO₂, CH₂Cl₂/MeOH 1:1) 0,7
MS (DCl) 183 (M+H)

### c) 2-Amino-3-nitro-berzoesäuremethylester

18 g (99 mmol) der Verbindung aus Referenzbeispiel 1b) werden in 200 ml Methanol mit 20 ml Thionylchlorid 48 h unter Rückfluß gerührt. Die Reaktionslösung wird im Rotationsverdampfer eingedampft, der Rückstand in 400 ml ges. Na₂CO₃-Lösung aufgenommen, 3x mit EE extrahiert, die vereinten organischen Phasen mit verdünnter Na₂CO₃- und ges. NaCl-Lösung gewaschen, über Na₂SO₄ getrocknet und eingeengt. Chromatographie an SiO₂ mit EE/Hep 9:1 und 7:3 liefert 11,5 g der Titelverbindung.
Schmp.: 86-88°C
R_{f} (SiO₂, EE/Hep 1:1) = 0,5
MS(DCl) 197 (M+H)

### d) 2-[N-(n-Pentanoyl)amino]-3-nitro-benzoesäuremethylester

7 g (35,5 mmol) der Verbindung aus Referenzbeispiel 1c) werden in 50 ml Valeriansäurechlorid 1h bei 110°C gerührt. Es wird zur Trockene eingeengt, der Rückstand in Ether 30 Min. mit Aktivkohle behandelt, filtriert, eingeengt und chromatographisch an SiO₂ mit EE/Hep 2:8 gereinigt. Es resultieren 5,8 g der Titelverbindung.
Schmp.: 66-69°C
R_{f} (SiO₂, EE/Hep 1:1) = 0,4
MS (DCl): 281 (M+H)

### e) 6-Bromomethyl-3-chlor-2-methoxycarbonyl-benzo[b]-thiophen

2,5 g (10,4 mmol) 3-Chlor-2-methoxycarbonyl-6-methylbenzo[b]thiophen (hergestellt nach J. Org. Chem. 41, 3399 (1976)) werden in 150 ml Chlorbenzol mit 1,87 NBS und 420 mg Di-benzoylperoxid 5h am Rückfluß gekocht. Nach Abdestillieren des Chlorbenzols im Rotationsverdampfer wird der erhaltene Rückstand in EE aufgenommen, die EE-Lösung mit ges. NaHCO₃-, 10 %iger Na₂SO₃- und ges. NaCl-Lösung gewaschen, über Na₂SO₄ getrocknet und eingeengt. Chromatographie an SiO₂ mit EE/Hep 1:20 liefert 2,28 g der Titelverbindung.
Schmp.: 143-145°C
R_{f} (SiO₂, EE/Hep 1:20) = 0,3, - MS (DCl): 319, 321 (M+H)

### f) 2-[N-(n-Pentanoyl)-((3-chlor-2-methoxycarbonyl-benzo[b]-thiophen-6-yl)methyl)]amino-3-nitro-benzoesäuremethylester

800 mg (2,86 mmol) der Verbindung aus Referenzbeispiel 1d) werden in 5 ml abs. DMF gelöst, mit 395 mg K₂CO₃ versetzt und die Mischung 10 Min. bei Raumtemperatur gerührt. Es wird eine Lösung von 913 mg der Verbindung aus Beispiel 1e) in 20 ml abs. DMF zugetropft und die Reaktions-Lösung über Nacht bei Zimmertemperatur gerührt. Das DMF wird dann im Vakuum abgezogen, der Rückstand in EE aufgenommen, die EE-Phase mit H₂O, verdünnter, gesättigter NaHCO₃ und gesättigter NaCl-Lösung gewaschen, über Na₂SO₄ getrocknet und eingeengt Chromatographie an SiO₂ mit EE/Hep 1:2 liefert 860 mg der Titelverbindung.
R_{f} (SiO₂, EE/Hep 1:2) = 0,3
MS(FAB): 519 (M+H)

### g) 2-n-Butyl-1-[(3-chlor-2-methoxycarbonyl-benzo[b]thiophen-6-yl). methyl]-1H-benzimidazol-7-carbonsäuremethylester

450 mg (0,85 mmol) der Verbindung aus Referenzbeispiel 1f) werden in 50 ml Ethanol 1h in Gegenwart von Raney-Nickel hydriert. Es wird der Katalysator abfiltriert, das Filtrat zur Trockene eingeengt und der resultierende Rückstand in 10 ml EE/Isopropanol (1:1) und 10 ml einer mit HCI gesättigten EE-Lösung 30 Min. bei 50°C gerührt. Nach Einengung und Kristallisation aus Methanol resultiert 190 mg der Titelverbindung.
Schmp.: 167-170°C (Zers.)
R_{f} (SiO₂, CH₂Cl₂/MeOH/NH₄OH 49/1/0,1) = 0,3
MS (DCl): 471 (M+H)

### h) 2-n-Butyl-1-[(2-carboxy-3-chlor-benzo[b]thiophen-6-yl)-methyl]-1Hbenzimidazol-7-carbonsäure

185 mg (0,39 mmol) der Verbindung aus Referenzbeispiel 1g) werden in 10 ml Ethanol gelöst, 1 ml H₂O und 1 ml konz. NaOH zugefügt und die erhaltene Lösung 3 h bei Raumtemperatur gerührt. Das EtOH wird im Vakuum abgezogen, die wäßrige Lösung auf einen pH von 3 mit Eisessig eingestellt und der ausfallende Niederschlag abgesaugt Nach Trocknung im Hochvakuum erhält man 100 mg der Titelverbindung in Form weißer Kristalle.
Schmp.: > 260°C
R_{f} (SiO₂, EE/MeOH 2/1) = 0,18
MS (FAB): 443 (M+H)

### Referenzbeispiel 2

### 2-n-Butyl-3-[(2-carboxy-3-chlor-benzo[b]thiophen-6-yl)methyl]-3Himidazo[4,5-b]pyridin

### a) 2-n-Butyl-3H-imidazo[4,5-b]pyridin

10 g (91,6 mmol) 2,3-Diaminopyridin und 27,4 g Valeriansäure werden 18 h bei 170°C gerührt. Nach Ende der Reaktion wird mit 100 ml CH₂Cl₂ verdünnt, mit gesättigter NaHCO₃-Lösung Wasser und gesättigter NaCl-Lösung gewaschen, über Na₂SO₄ getrocknet und eingeengt. Chromatographie an SiO₂ mit EE/Hep 20:1 liefert 9,7 g der Titelverbindung.
Schmp.: 103°C
R_{f} (SiO₂, EE/MeOH 20:1) = 0,3
MS (DCl): 176 (M+H)

### b) 2-n-Butyl-3-[(3-chlor-2-methoxycarbonyl-benzo[b]thiophen-6-yl)-methyl]-3H-imidazo[4,5-b]pyridin

300 mg (0,94 mmol) der Verbindung aus Referenzbeispiel 1 e) und 175 mg der Verbindung aus Beispiel 4 a) werden mit 552 mg K₂CO₃ in 10 ml DMF 8 h bei Raumtemperatur gerührt. Es wird zur Trockene eingeengt, der Rückstand in EE aufgenommen, die EE-Lösung mit H₂O, verdünnter KHSO₄-, gesättigter NaHCO₃- und gesättigter NaCI-Lösung gewaschen, über Na₂SO₄ getrocknet und eingeengt. Chromatographie an SiO₂ mit EE/Hep 1:1 liefert 130 mg der Titelverbindung als schwach gelbes Pulver.
Schmp.: 127-129°C
R_{f} (SiO₂, EE/Hep 1:1) = 0,2
MS (DCl): 414 (M+H)

### c) 2-n-Butyl-3-[(2-carboxy-3-chlor-benzo[b]thiophen-6-yl)methyl]-3Himidazo[4,5-b]pyridin

117 mg (0,28 mmol) der Verbindung aus Referenzbeispiel 2b) werden nach dem in Beispiel 1 h) angeführten Verfahren umgesetzt. Es resultieren 107 mg der Titelverbindung als weißes Pulver.
Schmp.: > 260°C
R_{f} (SiO₂, EE/MeOH 2:1) = 0,3
MS (FAB): 400 (M+H)

### Referenzbeispiel 3

### 2-n-Butyl-1-[(2'-ethoxycarbonylaminosulfonyl-biphenyl-4-yl)methyl]-1H-benzimidazol-7-carbonsäure

### a) 2-[N-(n-Pentanoyl)-((2'-N,N-dimethylaminoformyisulfonamido-biphenyl-4-yl)methyl)]amino-3-nitro-benzoesäuremethylester

7,9 g (28,2 mmol) der Verbindung aus Referenzbeispiel 1d) werden mit 10,7 g (28,2 mmol) der Verbindung aus Beispiel 12d) und 11,7 g (84,6 mmol) K₂CO₃ in 200 ml abs. DMF 24h bei Raumtemperatur gerührt. Anschließend wird zur Trockene eingeengt, der Rückstand in EE aufgenommen, die EE-Lösung 3x mit H₂O, 1x mit KHSO₄-Lösung (25 %ig), 1x mit gesättigter NaNCO₃-Lösung und 1x mit gesättigter NaCl-Lösung gewaschen, über MgSO₄ getrocknet und eingeengt. Der ölige Rückstand liefert nach Kristallisation aus EE/Diisopropylether 7,9 g der Titelverbindung. Chromatographie der eingeengten Mutterlauge an SiO₂ mit n-Heptan/EE (2/3) lieferte weitere 2,54 g der Titelverbindung.
Schmp.: 148-152°C
R_{f}(SiO₂, n-Heptan/EE 2:8) = 0,33
MS(FAB): 581 (M+H)

### b) 2-[N-(n-Pentanoyl)-((2'-N,N-dimethylaminoformylsulfonamido-biphenyl-4-yl)methyl]amino-3-amino-benzoesäuremethylester

10,4 g (17,9 mmol) der Verbindung aus Referenzbeispiel 3a) werden in 800 ml Methanol 3h in Gegenwart von Raney-Nickel hydriert. Der Katalysator wird abfiltriert, das Filtrat zur Trockene eingeengt und der Rückstand im Hochvakuum getrocknet, Es resultieren 9,9 g der Titelverbindung als amorpher Schaum.
R_{f}(SiO₂, CH₂Cl₂/MeOH 95:5) = 0,3
MS(FAB): 551 (M+H)

### c) 2-n-Butyl-1-[(2'-sulfonamidobiphenyl-4-yl)methyl]-1H-benzimidazol-7-carbonsäuremethylester

9,8 g (17,8 mmol) der Verbindung aus Referenzbeispiel 3h) werden in 180 ml Methanol mit 90 ml konzentrierter Salzsäure 3h am Rückfluß gerührt. Das Lösungsmittel wird verdampft, die verbleibende Lösung mit 6N NaOH-Lösung auf pH ∼ 5-6 eingestellt, die wäßrige Lösung 3x mit CH₂Cl₂ extrahiert, die vereinten organischen Phasen mit gesättigter NaCl-Lösung gewaschen und über MgSO₄ getrocknet Umkristallisation aus EE lieferte 8,16 g der Titelverbindung in Form weißer Kristalle.
Schmp.: 192-195°C
R_{f}(SiO₂, EE/n-Heptan 8:2) = 0,38
MS(FAB) = 478 (M+H)

Alternativ resultiert die Titelverbindung auch aus der Verbindung aus Referenzbeispiel 3a) nach diesem Verfahren. Dabei erhält man aus 100 mg (0,19 mmol) der Verbindung aus Referenzbeispiel 3 a) 60 mg der gewünschten Verbindung.

### d) 2-n-Butyl-1-((2'-dimethylaminoformylsulfonamido-biphenyl-4-yl)methyl]-1Hbenzimidazol-7-carbonsäuremethylester

150 mg (0,18 mmol) der Verbindung aus Referenzbeispiel 13b) werden unter Argon in 10 ml Isopropanol/EE (1:1) mit 10 ml einer HCl-gesättigten EE-Lösung über Nacht bei Raumtemperatur stehengelassen. Es wird eingeengt, der Rückstand in CH₂Cl₂ aufgenommen, die CH₂Cl₂-Phase mit gesättigter Na₂-CO₃-Lösung, Wasser und gesättigter NaCl-Lösung gewaschen und über MgSO₄ getrocknet. Einengung und Trocknung im Hochvakuum liefert 138 mg der Titelverbindung als amorphen Schaum.
R_{f}(SiO₂, CH₂Cl/MeOH 95:5) = 0,5
MS(FAB): 533 (M+H)

### e) 2-n-Butyl-1-[(2'-ethoxycarbonylaminosulfonyl-biphenyl-4-yl)-methyl]-1Hbenzimidazol-7-carbonsäuremethylester

3,25 g (6,81 mmol) der Verbindung aus Referenzbeispiel 3c) und 170 mg (1,36 mmol) DMAP werden in 12 ml absolutem Pyridin unter Argon bei 0°C mit 1,53 g (13,6 mmol) K-tert.-Butylat und nach 10 Minuten Rühren bei der gleichen Temperatur mit 0,65 ml (6,81 mmol) Chlorameisensäureethylester versetzt. Es wird über Nach bei Raumtemperatur gerührt. Anschließend wird die Lösung unter Eiskühlung mit einer 25 %igen KHSO₄-Lösung bis zur sauren Reaktion eingestellt und mehrfach mit EE extrahiert. Die vereinten organischen Phasen werden mit gesättigter NaCl-Lösung gewaschen, über MgSO₄ getrocknet und eingeengt Chromatographie an SiO₂ mit CH₂Cl₂/MeOH/NH₃ (9:1:0,1) lieferte 1,8 g der Titelverbindung als amorphen Schaum.
R_{f}(SiO₂; CH₂Cl₂/MeOH/HOAC 9:1:0,2) = 0,71
MS(FAB): 550 (M+H)

### f) 2-n-Butyl-1-[(2'-ethoxycarbonylaminosulfonyl-biphenyl-4-yl)methyl]-1Hbenzimidazol-7-carbonsäure

Die Herstellung der Titelverbindung aus der Verbindung aus Referenzbeispiel 19e) erfolgt nach dem in Referenzbeispiel 1h) angegebenen Verfahren.
R_{f}(SiO₂, CH₂Cl₂/MeOH/HOAC 9:1:0,2) = 0,64
MS(FAB): 536 (M+H)

### Referenzbeispiel 4

### 2-n-Butyl-1-[(2'-n-propylaminocarbonylaminosulfonyl-biphenyl-4-yl)methyl]-1Hbenzimidazol-7-carbonsäure

### a) 2-n-Butyl-[(2'-n-propylaminocarbonylaminosulfonyl-biphenyl-4-yl)methyl]-1H-benzimidazol-7-carbonsäuremethylester

100 mg (0,21 mmol) der Verbindung aus Referenzbeispiel 3c) werden in 8 ml absolutem Aceton mit 90 mg (0,6 mmol) K₂CO₃ in 24 µl (0,25 mmol) n-Propylisocyanat 2h am Rückfluß gekocht. Nach Abkühlung wird die Lösung durch Zusatz von 2N HCl auf pH ∼ 1 eingestellt und mehrfach mit CH₂Cl₂ extrahiert. Die vereinten organischen Phasen werden 1x mit H₂O und 1x mit gesättigter NaCl-Lösung gewaschen, über MgSO₄ getrocknet und eingeengt. Umkristallisation aus EE liefert 107 mg der Titelverbindung
Schmp.: 150-152°C
R_{f}(SiO₂, CH₂Cl₂/MeOH/NH₃ 9:1:0.2) = 0,24
MS(FAB): 563 (M+H)

### b) 2-n-Butyl-1-[(2'-n-propylaminocarbonylaminosulfonyl-biphenyl-4-yl)-methyl]-1H-benzimidazol-7-carbonsäure

Die Titelverbindung wird aus der Verbindung aus Referenzbeispiel 4a) nach dem in Referenzbeispiel 1h) angeführten Verfahren hergestellt. Aus 38 mg (0,07 mmol) 20 a) erhält man 30 mg der gewünschten Verbindung als amorphen Schaum.
R_{f}(SiO₂, CH₂Cl₂/MeOH/ACOH 9:1:0,2) = 0,2
MS(FAB): 549 (M+H)

### Beispiel 1

### 3-[(2'-Aminoethylphenyl)carbonylaminosulfonyl-biphenyl-4-yl)-methyl]-5,7-dimethyl-2-ethyl-3H-imidazo[4,5-b]pyridin

### a) Sulfonamidobrombenzol

51,6 g (0,3 mol) o-Bromanilin werden unter Argon-Atmosphäre zu einer Lösung aus 100 ml konz. HCl und 30 ml Eisessig gegeben, bei -10°C eine Lösung von 22,4 g Natriumnitrit in 30 ml Wasser zugetropft und die Reaktionslösung 60 Min bei -5°C gerührt. Die erhaltene Lösung wird zu einer mit SO₂ gesättigten Lösung von 7 g CuCl₂ x 2H₂O und 0,5 g CuCl in 300 ml Eisessig getropft, die Mischung nach 60 Min Rühren bei Raumtemperatur in ein Eis/Wasser-Gemisch gegossen, mit Ether extrahiert, die Ether-Extrakte mit ges. NaHCO₃-Lösung und Wasser gewaschen, über MgSO₄ getrocknet und eingeengt Die erhaltenen 67,8 g Sulforylchloridverbindung werden in 500 ml Aceton unter Kühlung mit 300 ml konz. Ammoniak versetzt. Nach Abzug des Acetons wird die resultierende Suspension mit Wasser verdünnt, die ausfallenden weißen Kristalle abgesaugt, mit H₂O gewaschen und im Hochvakuum getrocknet. Die Titelverbindung wird ohne weitere Reinigung in der folgenden Reaktion eingesetzt.

### b) 2-N,N-Dimethylaminoformylsulfonamidobrombenzol

0,236 mol der Verbindung aus Beispiel 1 a) werden in 150 ml abs. DMF mit 40 ml N,N-Dimethylformamiddimethylacetal 2 h bei Raumtemperatur gerührt. Die Reaktionslösung wird auf 200 ml 5 %ige NaHSO₄-Lösung/Eis (1:1) gegossen, der ausfallende Niederschlag abgesaugt, mit H₂O gewaschen und im Vakuum getrocknet. Man erhält 67 g der Titelverbindung.
R_{f} (SiO₂, EE/Hep 1:1) = 0,1
MS (DCl): 291/293 (M+H)

### c) 4'-Methyl-biphenyl-2-N,N-dimethylaminoformylsulfonamid

Zu 11 g (37,9 mmol) der Verbindung aus Beispiel 1 b), 1 g Triphenylphosphin, 8 g Na₂CO₃ in 150 ml Toluol und 40 ml H₂O gibt man unter Argon zuerst 420 mg Pd(OAC)₂ und anschließend 5,66 g (41,9 mmol) Tolylboronsäure in 100 ml Ethanol. Nun wird 4 h zum Sieden erhitzt, dann eingeengt und in 500 ml EE und 500 ml H₂O aufgenommen. Der entstehende Niederschlag wird abfiltriert und als Titelverbindung charakterisiert. Die EE-Phase wird abgetrennt, über Na₂SO₄ getrocknet und eingeengt. Chromatographie an SiO₂ mit EE liefert einen weiteren Anteil der Titelverbindung; Gesamtausbeute: 7,6 g
R_{f} (SiO₂, EE/Hep 1:1) = 0,2
MS (DCl): 303 (M+H)

### d) 4'-Brommethylbiphenyl-2-N,N-dimethylamino-formylsulfonamid

Die Titelverbindung wird aus der Verbindung 1 c) nach dem Verfahren des Referenzbeispiels 1 e) hergestellt Dabei resultieren aus 3,8 g (13,5 mmol) der Verbindung 1 c) 1,2 g der Titelverbindung.
R_{f} (SiO₂, EE/Hep 2:1) = 0,2
MS (DCI): 381/383 (M+H)

### e) 5,7-Dimethyl-3-[(2'-N,N-dimethylaminoformylsulfonamido-biphenyl-4-yl)-methyl]-2-ethyl-3H-imidazo[4,5-b]pyridin

Die Titelverbindung wird aus der Verbindung des Beispieles 12 d) und 5,7-Dimethyl-2-ethyl-3H-imidazo[4,5-b]pyridin nach dem Verfahren des Beispiels 9 a) hergestellt. Man erhält aus 3,2 g der Verbindung 1 d) 1,1 g der Titelverbindung.
R_{f} (SiO₂, EE/MeOH 10:1) = 0,2
MS (FAB): 476 (M+H)

### f) 5,7-Dimethyl-2-ethyl-3-[(2'-sulfonamidobiphenyl-4-yl)methyl]-3H-imidazo[4,5-b]pyridin

0,6 g (1,26 mmol) der Verbindung aus Beispiel 1 e) werden in 20 ml Ethanol mit 10 ml konz. HCl-Lösung 45 Min. am Rückfluß gekocht Das Ethanol wird im Vakuum entfernt, mit gesättigter NaHCO₃-Lösung neutralisiert, mit NaHSO₄-Lösung auf pH - 5-6 eingestellt und mit EE extrahiert. Die EE-Phase wid getrocknet (Na₂SO₄) und eingeengt, wobei man 380 mg der Titelverbindung erhält.
R_{f} (SiO₂, EE/Hep 5:1) = 0,5
MS (FAB): 421 (M+H)

### g) 5,7-Dimethyl-2-ethyl-3-[(2'-ethoxycarbonylaminosulfonylbiphenyl-4-yl)-methyl]-3H-imidazo[4,5-b]pyridin

0,52 g (1,2 mmol) der Verbindung aus Beispiel 1 f) und 340 mg K₂CO₃ werden unter Argon in 10 ml trockenem DMF mit 266 mg (2,4 mmol) Chlorameisensäureethylester 3 h zum Rückfluß erhitzt. Nach Abkühlung auf Zimmertemperatur wird mit 10 % NaHSO₄ versetzt, mit EE extrahiert und die organische Phase über MgSO₄ getrocknet Einengung und Chromatographie an SiO₂ mit EE als Eluent liefert 250 mg der Titelverbindung.
R_{f} (SiO₂, EE) = 0,2
MS (FAB): 493 (M+H)

### h) 3-[(2'-(Aminoethytphenyl)carbonylaminosulfonyl-biphenyl-4-yl)-methyl]-5,7-dimethyl-2-ethyl-3H-imidazo[4,5-b]pyridin

80 mg (0,16 mmol) der Verbindung aus Beispiel 1 g) und 50 µl Phenylethylamin werden in 5 ml abs. Toluol unter Argon 1,5 h am Rückfluß gekocht. Nach Einengung und Chromatographie an SiO₂ mit EE/MeOH 10:1 resultieren 70 mg der Titelverbindung nach Gefriertrocknung als amorphes Pulver.
R_{f} (SiO₂, EE/MeOH 10:1) = 0,4
MS (FAB): 568.(M+H)

### Beispiel 2

### 3-[(2'-(Aminomethylcyclohexyl)-carbonylaminosulfonyl-biphenyl-4-yl)-methyl]-5,7-dimethyl-2-ethyl-3H-imidazo[4,5-b]pyridin

Die Titelverbindung wird nach dem Verfahren des Beispiels 1 h) aus der Verbindung aus Beispiel 1 g) und Cyclohexylmethylamin hergestellt; es resultieren aus 80 mg (0,16 mmol) Beispiel 1 g) 90 mg der Titelverbindung nach Gefriertrocknung als amorpher Farbstoff.
R_{f} (SiO₂, EE) = 0,3
MS (FAB): 560 (M+H)

### Beispiel 3

### 3-[(2'-(Diallylamino)carbonylaminosulfonyl-biphenyl-4-yl)methyl]-5,7-dimethyl-2-ethyl-3H-imidazo[4,5-b]pyridin

Die Titelverbindung wird nach dem Verfahren des Beispiels 1 h) aus der Verbindung aus Beispiel 1 g) und Diallylamin hergestellt. Es resultieren 60 mg der Titelverbindung aus 80 mg (0,16 mmol) Beispiel 1 g) als amorpher Feststoff.
R_{f} (SiO₂, EE/MeOH 10:1) = 0,2
MS (FAB): 544 (M+H)

### Beispiel 4

### 3-[(2'-(N,N-Diallyloxycarbonyl)aminosulfonyl-biphenyl-4-yl)methyl]-5,7-dimethyl-2-ethyl-3H-imidazo[4,5-b]pyridin

100 mg (0,23 mmol) der Verbindung aus Beispiel 1 f) werden in 10 ml abs. DMF unter Argon mit 66 mg (0,46 mmol) K₂CO₃ und 57 mg (0,46 mmol) Chloramelsensäureallylester 45 Min. zum Sieden erhitzt. Nach Einengung, Aufnahme in EE, waschen der.EE-Phase mit 10 %iger Na₂KSO₄-Lösung, Trocknung (MgSO₄) und Chromatographie:an SiO₂ mit EE resultieren 70 mg der Titelverbindung nach Gefriertrocknung.
R_{f} (SiO₂, EE) = 0,6
MS (FAB): 589 (M+H)

### Beispiel 5

### 3-[(2'-(N,N-Dibenzyloxycarbonyl)aminosulfonyl-bihenyl-4-yl)methyl]-5,7-dimethyl-2-ethyl-3H-imidazo[4,5-b]pyridin

Diese Verbindung wird nach dem Verfahren des Beispieles 4 aus der Verbindung des Beispieles 1 f) und Chlorameisensäurebenzylester hergestellt. Es resultieren aus 100 mg (0,23 mmol) der Verbindung 1 f) 70 mg der Titelverbindung.
R_{f} (SiO₂, EE) = 0,2
MS (FAB): 689 (M+H)

### Beispiel 6

### 3-[(2'-(Cyclohexylmethoxycarbonyl)aminosulfonyl-biphenyl-4-yl)methyl]-5,7-dimethyl-2-ethyl-imidazo[4,5-b]pyridin

Die Titelverbindung wird aus der Verbindung des Beispieles 1 f) und Chlorameisensäurecyclohexylmethylester nach dem Verfahren des Beispieles 4 hergestellt, wobei Amid und Ester jedoch im äquimolarem Verhältnis eingesetzt werden.
R_{f} (SiO₂; Methyl-tert-butylether) = 0,2
MS (FAB): 561 (M+H)

### Beispiel 7

### 5,7-Dimethyl-2-ethyl-3-[(2'-ethyloxycarbonyl)aminosulfonyl-biphenyl-4-yl)methyl]-3Himidazo[4,5-b]pyridin

Die Titelverbindung resultiert aus der Verbindung des Beispieles 1 f) und Chlorameisensäureethylester nach dem Verfahren des Beispieles 6.
R_{f} (SiO₂,EE) = 0.2
MS (FAB): 493 (M+H)

### Beispiel 8

### 2-n-Butyl-3-[(2'-ethoxycarbonylaminosulfonyl-biphenyl-4-yl)methyl]-3H-imidazo[4,5-b]/[5,4-b]-pyridin

### a) 2-n-Butyl-3-[(2'-N,N-dimethylaminoformylsulfonamido-biphenyl-4-yl)methyl]-3H-imidazo-[4,5-b]/[5,4-b]-pyridin

Die Titelverbindung wird aus den Verbindungenaus den Referenzbeispiel 2a) und Beispiel 1d) nach dem in Referenzbeispiel 4b) beschriebenen Verfahren hergestellt. Die Reinigung erfolgte durch Chromatographie an SiO₂ mit EE/MeOH 20:1 als Laufmittel und Kristallisation aus EE/Diisopropylester.
Schmp.: 205-211°C
R_{f}(SiO₂, EE/MeOH 20:1) = 0,15
MS(FAB): 476 (M+H)

### b) 2-n-Butyl-3-[(2'-sulfonamidobiphenyl-4-yl)methyl]-3H-imidazo[4,5-b]/[5,4-b]-pyridin

Diese Verbindung wird aus der Verbindung aus 8a) nach dem Verfahren des Referenzbeispiels 3C) und Chromatographie an SiO₂ mit EE/MeOH 20:1 als Laufmittel hergestellt.
R_{f}(SiO₂, EE/MeOH 20:1) = 0,39
MS(FAB): 421 (M+H)

### c) 2-n-Butyl-3-[(2'-ethoxycarbonylaminosulfonyl-biphenyl-4-yl)methyl]-3H-imidazo-[4,5-b]/[(5,4-b]pyridin

1 g (2,38 mmol) der Verbindung aus Beispiel 8b) werden unter Argon in 25 ml absolutem Dimethoxyethan mit 1 g aktiviertem (Hochvakuumtrocknung bei 150°C für 3h) Molsieb 4 Å, 0,66 g K₂CO₃ und 232 µl Chlorameisensäureethylester 6h am Rückfluß erhitzt. Es wird nach Abkühlung mit 100 ml 10%iger KH₂PO₄-Lösung versetzt (pH ∼ 4), 3x mit EE extrahiert, die vereinten EE-Extrakte über Na₂SO₄ getrocknet und eingeengt. Chromatographie an SiO₂ (EE/MeOH 20:1) liefert 0,5 g der Titelverbindung.
Schmp.: 172°C
R_{f}(SiO₂, EE/MeOH 20:1) = 0,3
MS(FAB): 493 (M+H)

### Beispiel 9

### 2-n-Butyl-3-[(2'-isopropylaminocarbonylaminosulfonyl-biphenyl-4-yl)-methyl]-3H-imidazo[4,5-b]/[5,4-b]pyridin

Die Titelverbindung resultiert aus 100 mg (0,2 mmol) der Verbindung aus Beispiel 8c) nach 3h Rückflußkochen mit 209 µl (2,44 mmol) Isopropylamin in 5 ml Toluol, Einengung und Chromatographie an SiO₂ (EE) in einer Ausbeute von 45 mg. Schmp.: 113-114°C
R_{f}(SiO₂, EE/MeOH 20:1) = 0,36
MS(FAB): 506 (M+H)

### Beispiel 10

### 2-n-Butyl-3-[(2'-allylaminocarbonylaminosulfonyl-biphenyl-4-yl)-methyl]-3H-imidazo[4,5-b]/[5,4-b]pyridin

Die Titelverbindung resultiert aus dem Umsatz der Verbindung aus Beispiel 8b) mit Allylisocyanat analog zu dem in Referenzbeispiel 20a) beschriebenen Verfahren.
Schmp.: 121°C
R_{f}(SiO₂, EE/MeOH 20:1) = 0,26
MS(FAB): 504 (M+H)

### Beispiel 11

### 2-n-Butyl-3-[(2'-n-propylaminocarbonylaminosulfonyl-biphenyl-4-yl)-methyl]-3H-imidazo-[4,5-b]/[5,4-b]pyridin

150 mg (0,3 mmol) der Verbindung aus Beispiel 8c) werden in 5 ml Toluol mit 295 µl (3,6 mmol) n-Propytamin 3h am Rückfluß gekocht. Es wird eingeengt und der Rückstand an SiO₂ (EE) chromatographiert. Man erhielt 90 mg der Titelverbindung.
Schmp.: 137-138°C
R_{f}(SiO₂,EE) = 0,2
MS(FAB): 506 (M+H)

### Beispiel 12

### 2-n-Butyl-3-[(2'-benzyloxycarbonylaminosulfonyl-biphenyl-4-yl)methyl]-3H-imidazol[4,5-b][5,4-b]pyridin

Die Titelverbindung wird aus der Verbindung aus Beispiel 8b) und Chlorameisensäurebenzylester nach dem im Beispiel 8c) beschriebenen Verfahren hergestellt.
Schmp.: 85°C
R_{f}(SiO₂, EE/MeOH 20:1) = 0,29
MS(FAB): 555 (M+H)

### Beispiel 13

### 2-Ethyl-7-methyl-3-[(2'-n-propylaminocarbonylaminosulfonyl-biphenyl-4-yl)methyl]-imidazo-[4,5-b]-pyridin

### a) 2-Ethyl-7-methyl-3H-imidazo[4,5-b]pyridin

10 g (65,3 mmol) 2-Amino-4-methyl-3-nitropyridin werden in 40 ml Tetrahydrofuran und 40 ml Methanol in Gegenwart von Raney-Nickel hydriert. Der Katalysator wird abfiltriert, das Lösungsmittel abgezogen, der Rückstand mit ethanolischer HCI-Lösung versetzt und das ausfallende 2,3-Diamino-4-methylpyridin-hydrochlorid abgesaugt. 7 g dieses Hydrochlorids werden in 57 g Polyphosphorsäure (aus 28,5 g P₂O₅ und 28,5 g H₃PO₄ (85 %ig)) gelöst, mit 1,26 ml Propionsäure versetzt und die Lösung 2h bei 100°C gerührt. Nach Abkühlung wird auf Eiswasser gegossen, durch Na₂CO₃-Zusatz alkalisch gestellt und mehrfach mit EE extrahiert. Die vereinten EE-Phasen werden mit gesättigter NaCl-Lösung gewaschen, über Na₂SO₄ getrocknet, eingeengt und derRückstand an SiO₂ chromatographiert (EE/MeOH 5:1).Es resultieren 4,2 g der Titelverbindung.
R_{f}(SiO₂, EE/MeOH 5:1) 0,4
MS(DCl): 162 (M+H)

### b) 3-[(2'-N,N-Dimethylaminoformylsulfonamido-biphenyl-4-yl)methyl]-2-ethyl-7-methyl-imidazo[4,5-b]pyridin

3,1 g (19,26 mmol) der Verbindung aus Beispiel 13a) und 9,15g (19,26 mmol) der Verbindung aus Beispiel 1d) (75%ig) werden in 200 ml absolutem DMF in Gegenwart von 2,6g (19,6 mmol) K₂CO₃ über Nacht bei Raumtemperatur gerührt. Das Lösungsmittel wird anschließend abgezogen, der Rückstand in CH₂Cl₂ aufgenommen, die CH₂Cl₂-Lösung mit H₂O gewaschen, über Na₂SO₄ getrocknet und eingeengt. Chromatographie an SiO₂ (EE/MeOH) 20:1) liefert 2,8 g der Titelverbindung.
Schmp.: 168-170°C
R_{f} (EE/MeOH 20:1) = 0,13
MS(FAB): 462 (M+H)

### c) 2-Ethyl-7-methyl-3-[(2'-sulfonamidobiphenyl-4-yl)methyl]-imidazo[4,5-b]pyridin 2,8 g (6,06 mmol) der Verbindung aus Beispiel 13b) werden nach dem in Referenzbeispiel 3c) angeführten Verfahren in die Titelverbindung (2,2 g) überführt.

Schmp.: 211-212°C
R_{f}(SiO₂, EE/MeOH) = 0,35
MS(FAB): 407 (M+H)

### d) 2-Ethyl-7-methyl-3-[(2'-n-propylaminocarbonylaminosulfonyl-biphenyl-4-yl)methyl]-imidazo[4,5-b]pyridin

Die Titelverbindung wird aus der Verbindung aus Beispiel 13c) und n-Propylisocyanat nach dem Verfahren des Referenzbeispiels 4a) hergestellt. Aus 70 mg (0,172 mmol) Verbindung 13c) resultieren 43 mg des gewünschten Produkts. Schmp.: 215-220°C
R_{f}(SiOₐ, EE/MeOH 20:1) = 0,36
MS(FAB): 492 (M+H)

### Beispiel 14

### 2-Ethyl-3-[(2'-ethylaminocarbonylaminosulfonyl-biphenyl-4-yl)methyl]-7-methylimidazo[4,5-b]pyridin

Die Titelverbindung wird aus der Verbindung des Beispiels 13c) und Ethylisocyanat nach dem Verfahren des Referenzbeispiels 4a) hergestellt.
Schmp.: 240-245°C
R_{f}(SiO₂, EE) = 0,14
MS(FAB): 478 (M+H)

### Beispiel 15

### 3-[(2'-Allylaminocarbonylaminosulfonyl-biphenyl-4-yl)methyl]-2-ethyl-7-methylimidazo[4,5-b]pyridin

Die Herstellung der Titelverbindung erfolgt durch Umsatz der Verbindung aus Beispiel 13c) und Allylisocyanat nach dem Verfahren des Referenzbeispiels 4a).
Schmp.: 216-219°C
R_{f}(SiO₂, EE) = 0,13
MS(FAB): 490 (M+H)

### Beisiel 16

### 2-Ethyl-3-[(2'-methoxycarbonylaminosulfonyl-biphenyl-4-yl)methyl]-7-methylimidazo[4,5-b]pyridin

100 mg (0,245 mmol) der Verbindung aus Beispiel 13c) werden mit 171 mg (1,24 mmol) K₂CO₃, 62 µl (0,62 mmol) Dimethyldicarbonat und 25 mg DMAP in 10 ml Diethylenglycoldimethylether 2h unter Rückfluß gerührt. Anschließend wird abdestilliert, der Rückstand mit einer EE/KH₂PO₄-Lösung versetzt, die organische Phase abgetrennt und 2x mit einer KH₂PO₄-Lösung gewaschen. Trocknung über Na₂SO₄, Einengung und Chromatographie an SiO₂ (EE) lieferte 44 mg der Titelverbindung.
R_{f}(SiO₂, EE) = 0,15
MS(FAB): 465 (M+H)

Die in der folgenden Tabelle aufgeführten Beispiele der Formel V wurden analog zu den in den Referenzbeispielen 1-4 und in den Beispielen 1-16 angeführten Vorschriften aus den beschriebenen Bausteinen hergestellt:

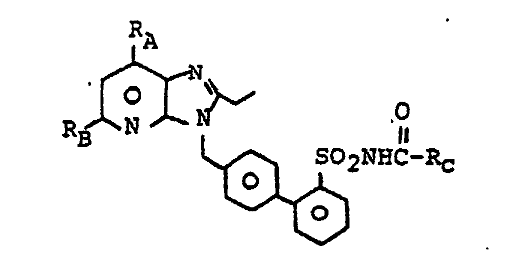

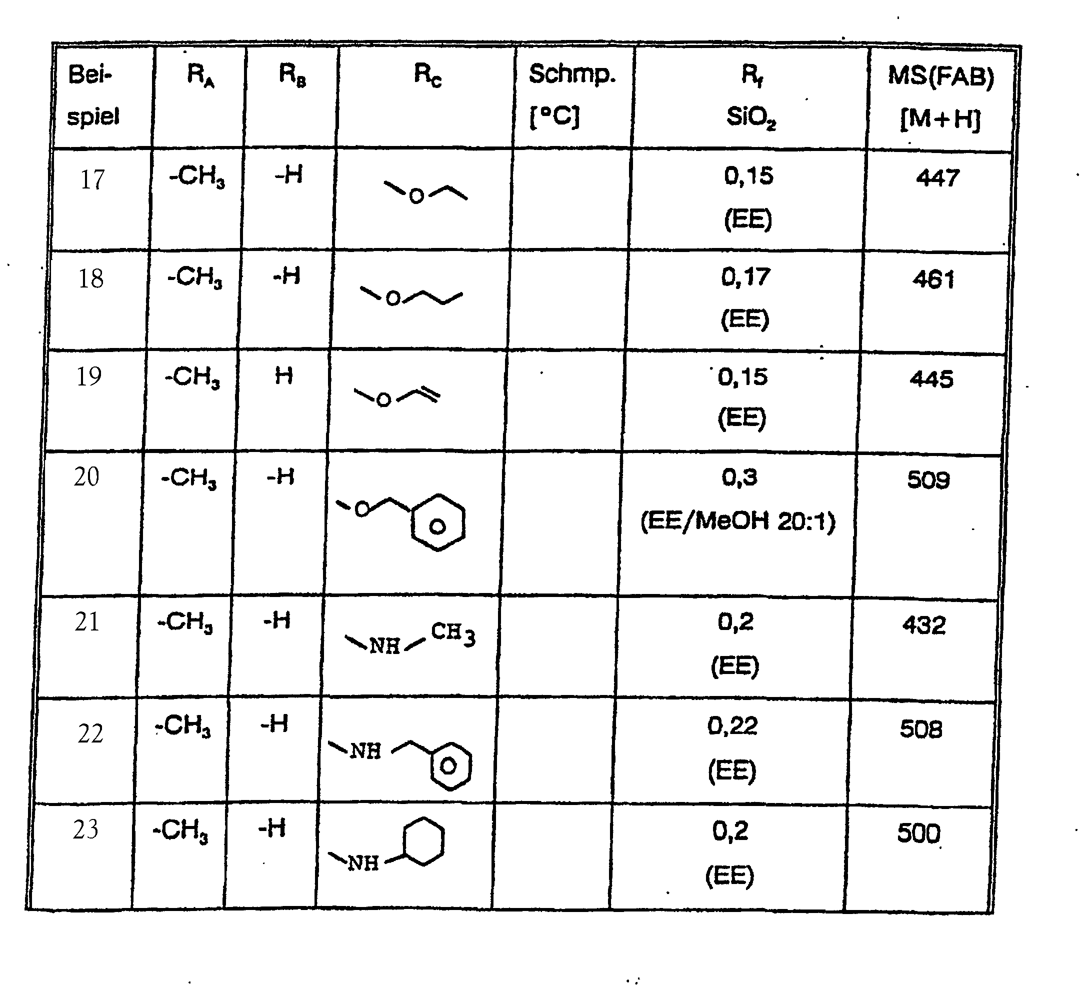

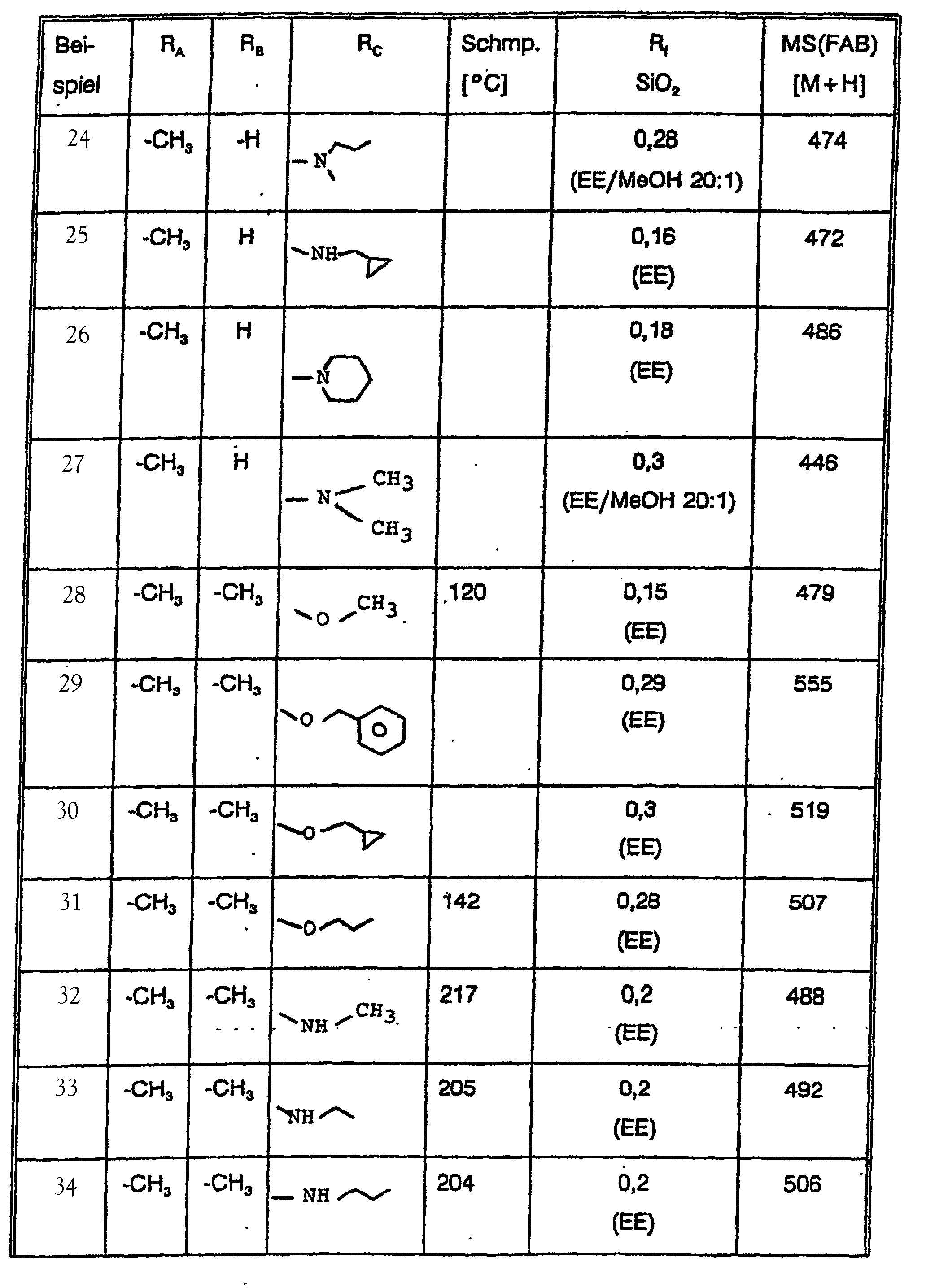

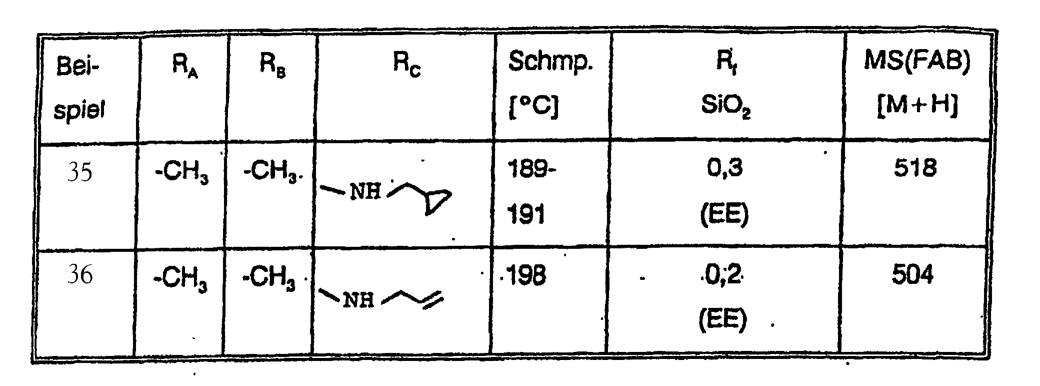

## Patentansprüche (Patentansprüche für folgende(n) Vertragsstaat(en): AT, BE, CH, LI, DE, DK, FR, GB, IT, LU, NL, PT, SE)

1. Verbindung der Formel (I) 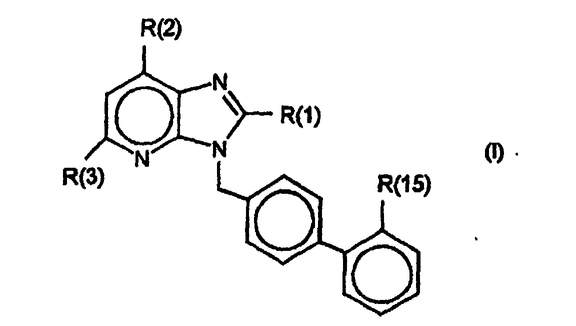 worin bedeuten
a)
R(1) Ethyl;
R(2) Methyl;
R(3) Wasserstoff;
R(15)
1. - SO₂NH-CO-O-R(17) mit
R(17) gleich Methyl, Ethyl, Propyl, Vinyl oder Benzyl;
2. - SO₂-NH-CO-NH-R(17) mit
R(17) gleich Methyl, Ethyl, Propyl, Benzyl, Cyclohexyl, Cyclopropylmethyl oder Allyl;
3. - SO₂-NH-CO-N(CH₃)-R(17) mit
R(17) gleich Methyl oder Propyl; oder
4. 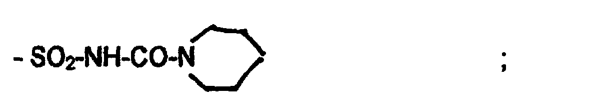
b)
R(1) Ethyl;
R(2) Methyl;
R(3) Methyl;
R(15)
1. - SO₂-NH-CO-O-R(17) mit
R(17) gleich Methyl, Ethyl, Propyl, Cyclopropylmethyl, Benzyl oder Cyclohexylmethyl;
2. - SO₂NH-CO-NH-R(17) mit
R(17) gleich Methyl, Ethyl, Propyl, Allyl, Cyclopropylmethyl, Cyclonexylmethyl oder Phenylethyl;
3. SO₂NH-CO-N(Allyl)₂;
4. SO₂N-(CO-O-Allyl)₂; oder
5. SO₂N-(CO-O-Benzyl)₂;
c)
R(1) n-Butyl;
R(2) Wasserstoff;
R(3) Wasserstoff;
R(15)
1. -SO₂-NH-CO-O-R(17) mit
R(17) gleich Ethyl oder Benzyl; oder
2. -SO₂NH-CO-NH-R(17) mit
R(17) gleich n-Propyl; iso-Propyl oder Allyl;
sowie deren physiologisch verträglichen Salze.

2. Verfahren zur Herstellung einer Verbindung der Formel I nach Anspruch 1, **dadurch gekennzeichnet, daß** man Verbindungen der Formel II 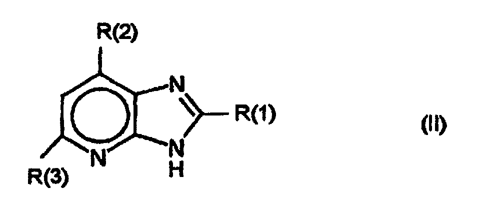 worin R(1), R(2) und R(3) wie oben definiert sind, alkyliert mit Verbindungen der Formel III 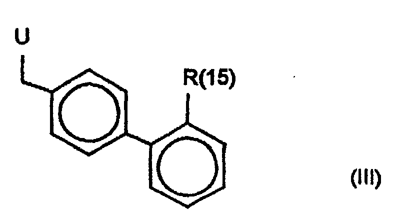 worin R(15) wie oben definiert ist und U für eine Fluchtgruppe steht, gegebenenfalls eingeführte Schutzgruppen wieder abgespaltet und die erhaltenen Verbindungen der Formel I gegebenenfalls in ihre physiologisch verträglichen Salze überführt.

3. Verbindung der Formel I nach Anspruch 1 zur Verwendung als Arzneimittel.

4. Verwendung einer Verbindung der Formel I nach Anspruch 1 zum Herstellen eines Medikaments mit Angiotensin-II-Rezeptoren-antagonistischer Wirkung.

## Patentansprüche (Patentansprüche für folgende(n) Vertragsstaat(en): ES, GR)

1. verfahren zur Herstellung einer Verbindung der Formel (I) 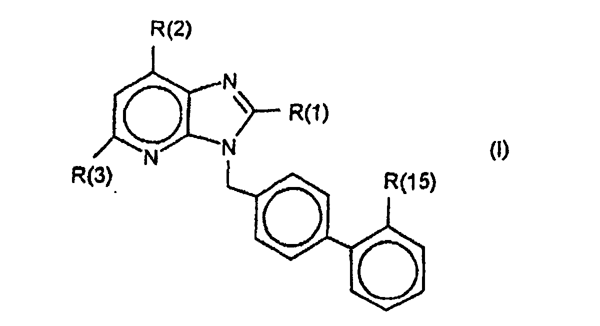 worin bedeuten
a)
R(1) Ethyl;
R(2) Methyl;
R(3) Wasserstoff;
R(15)
1. - SO₂-NH-CO-O-R(17) mit
R(17) gleich Methyl, Ethyl, Propyl, Vinyl oder Benzyl;
2. - SO₂NH-CO-NH-R(17) mit
R(17) gleich Methyl, Ethyl, Propyl, Benzyl, Cyclohexyl, Cyclopropylmethyl oder Allyl;
3. - SO₂-NH-CO-N(CH₃)-R(17) mit
R(17) gleich Methyl oder Propyl; oder
4. - SO₂-NH-CO-N ;
b)
R(1) Ethyl;
R(2) Methyl;
R(3) Methyl;
R(15)
1. - SO₂-NH-CO-O-R(17) mit
R(17) gleich Methyl, Ethyl, Propyl, Cyclopropylmethyl, Benzyl oder Cyclohexylmethyl;
2. - SO₂-NH-CO-NH-R(17) mit
R(17) gleich Methyl, Ethyl, Propyl, Allyl, Cyclopropylmethyl, Cyclohexylmethyl oder Phenylethyl;
3. SO₂NH-CO-N(Allyl)₂;
4. SO₂N-(CO-O-Allyl)₂; oder
5. SO₂N-(CO-O-Benzyl)₂;
c)
R(1) n-Butyl;
R(2) Wasserstoff;
R(3) Wasserstoff;
R(15)
1. -SO₂-NH-CO-O-R(17) mit
R(17) gleich Ethyl oder Benzyl; oder
2. -SO₂-NH-CO-NH-R(17) mit
R(17) gleich n-Propyl; iso-Propyl oder Allyl;
sowie deren physiologisch verträglichen Salze,
**dadurch gekennzeichnet, daß** man Verbindungen der Formel II 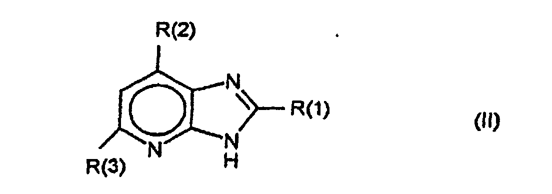 worin R(1), R(2) und R(3) wie oben definiert sind, alkyliert mit Verbindungen der Formel III 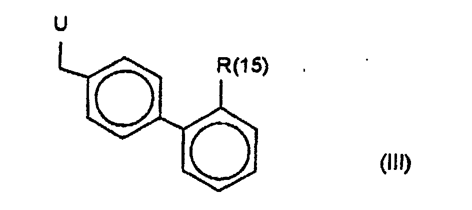 worin R(15) wie oben definiert ist und U für eine Fluchtgruppe steht, gegebenenfalls eingeführte Schutzgruppen wieder abgespaltet und die erhaltenen Verbindungen der Formel I gegebenenfalls in ihre physiologisch verträglichen Salze überführt.

2. Verwendung einer Verbindung der Formel I nach Anspruch 1 zum Herstellen eines Medikaments mit Angiotensin-II-Rezeptoren-antagonistischer Wirkung.

## Claims (Claims for the following Contracting State(s): AT, BE, CH, LI, DE, DK, FR, GB, IT, LU, NL, PT, SE)

1. A compound of the formula (I) 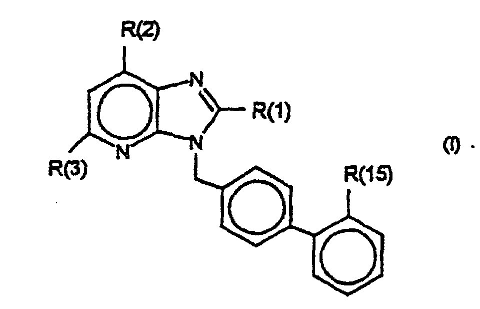 in which
a)
R(1) is ethyl;
R(2) is methyl;
R(3) is hydrogen;
R(15) is
1. -SO₂-NH-CO-O-R(17), with
R(17) being methyl, ethyl, propyl, vinyl or benzyl;
2. -SO₂-NH-CO-NH-R(17), with
R(17) being methyl, ethyl, propyl, benzyl, cyclohexyl, cylcopropylmethyl or allyl;
3. -SO₂-NH-CO-N(CH₃)-R(17), with
R(17) being methyl or propyl; or
4. 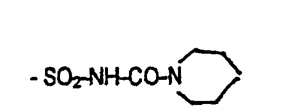
b)
R(1) is ethyl;
R(2) is methyl;
R(3) is methyl;
R(15) is
1. -SO₂-NH-CO-O-R(17), with
R(17) being methyl, ethyl, propyl, cyclopropylmethyl, benzyl or cyclohexylmethyl;
2. -SO₂-NH-CO-NH-R(17), with
R(17) being methyl, ethyl, propyl, allyl, cyclopropylmethyl, cyclohexylmethyl or phenylethyl;
3 . SO₂NH-CO-N(allyl)₂;
4. SO₂N-(CO-O-allyl)₂; or
5. SO₂N- (CO-O-benzyl)₂;
c)
R(1) is n-butyl;
R(2) is hydrogen;
R(3) is hydrogen;
R(15) is
1. -SO₂-NH-CO-O-R(17), with
R(17) being ethyl or benzyl; or
2. -SO₂-NH-CO-NH-R(17), with
R(17) being n-propyl; isopropyl or allyl;
and its physiologically tolerable salts.

2. A process for preparing a compound of the formula I as claimed in claim 1, which comprises alkylating compounds of the formula II 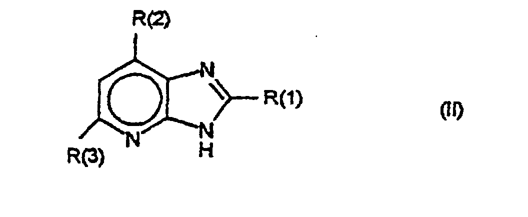 in which R(1), R(2) and R(3) are as defined above, with compounds of the formula III 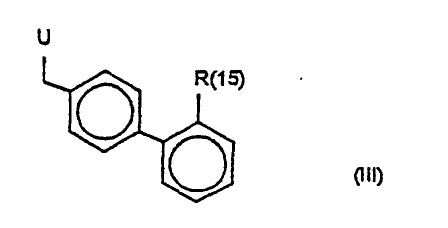 in which R(15) is as defined above and U is a leaving group, optionally removing introduced protective groups again and optionally converting the compounds of the formula I obtained into their physiologically tolerable salts.

3. A compound of the formula I as claimed in claim 1 for use as a medicament.

4. The use of a compound of the formula I as claimed in claim 1 for preparing a medicament having angiotensin II receptor antagonistic action.

## Claims (Claims for the following Contracting State(s): ES, GR)

1. A process for preparing a compound of the formula (I) 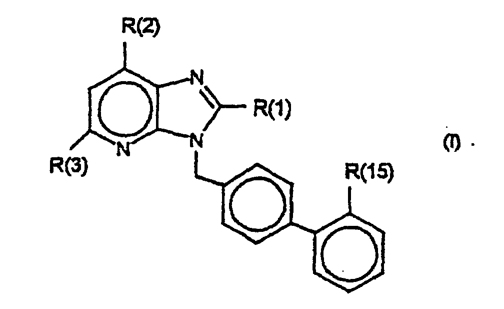 in which
a)
R(1) is ethyl;
R(2) is methyl;
R(3) is hydrogen;
R(15) is
1. -SO₂-NH-CO-O-R(17), with
R(17) being methyl, ethyl, propyl, vinyl or benzyl;
2. SO₂-NH-CO-NH-R(17), with
R(17) being methyl, ethyl, propyl, benzyl, cyclohexyl, cylcopropylmethyl or allyl;
3. -SO₂-NH-CO-N(CH₃)-R(17), with
R(17) being methyl or propyl; or
4. - SO₂-NH-CO-N
b)
R(1) is ethyl;
R(2) is methyl;
R(3) is methyl;
R(15) is
1. -SO₂-NH-CO-O-R(17), with
R(17) being methyl, ethyl, propyl, cyclopropylmethyl, benzyl or cyclohexylmethyl;
2. -SO₂-NH-CO-NH-R(17), with
R(17) being methyl, ethyl, propyl, allyl, cyclopropylmethyl, cyclohexylmethyl or phenylethyl;
3. SO₂NH-CO-N(allyl)₂;
4. SO₂N-(CO-O-allyl)₂; or
5. SO₂N-(CO-O-benzyl)₂;
c)
R(1) is n-butyl;
R(2) is hydrogen;
R(3) is hydrogen;
R(15) is
1. -SO₂-NH-CO-O-R(17), with
R(17) being ethyl or benzyl; or
2. -SO₂-NH-CO-NH-R(17), with
R(17) being n-propyl; isopropyl or allyl;
and its physiologically tolerable salts, which comprises alkylating compounds of the formula II 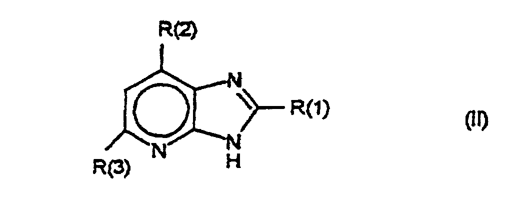 in which R(1), R(2) and R(3) are as defined above, with compounds of the formula III 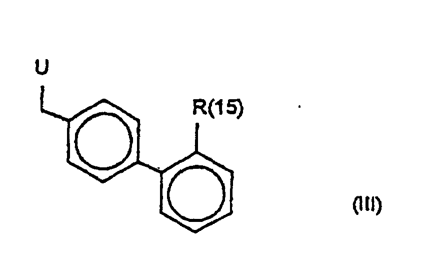 in which R(15) is as defined above and U is a leaving group, optionally removing introduced protective groups again and optionally converting the compounds of the formula I obtained into their physiologically tolerable salts.

2. The use of a compound of the formula I as claimed in claim 1 for preparing a medicament having angiotensin II receptor antagonistic action.

## Revendications (Revendications pour l'(les) Etat(s) contractant(s) suivant(s): AT, BE, CH, LI, DE, DK, FR, GB, IT, LU, NL, PT, SE)

1. Composé de formule (I) 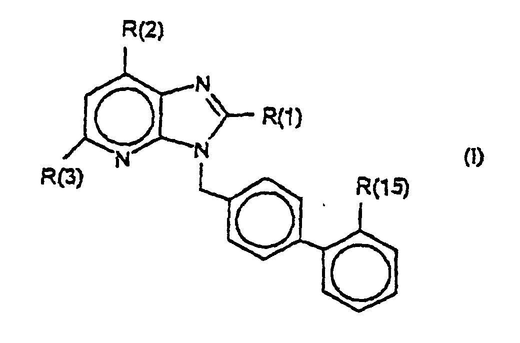 dans laquelle
a)
R(1) représente un groupe éthyle;
R(2) représente un groupe méthyle;
R(3) représente un atome d'hydrogène;
R(15) représente
1. un groupe -SO₂-NH-CO-O-R(17) dans lequel R(17) est un groupe méthyle, éthyle, propyle, vinyle ou benzyle;
2. un groupe -SO₂-NH-CO-NH-R(17) dans lequel R(17) est un groupe méthyle, éthyle, propyle, benzyle, cyclohexyle, cyclopropylméthyle ou allyle;
3. un groupe -SO₂-NH-CO-N(CH₃)-R(17) dans lequel R(17) est un groupe méthyle ou propyle; ou
4. un groupe 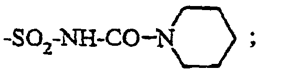
b)
R(1) représente un groupe éthyle;
R(2) représente un groupe méthyle;
R(3) représente un groupe méthyle;
R(15) représente
1. un groupe -SO₂-NH-CO-O-R(17) dans lequel R(17) est un groupe méthyle, éthyle, propyle, cyclopropylméthyle, benzyle ou cyclohexylméthyle;
2. un groupe -SO₂-NH-CO-NH-R(17) dans lequel R(17) est un groupe méthyle, éthyle, propyle, allyle, cyclopropylméthyle, cyclohexylméthyle ou phényléthyle;
3. un groupe SO₂NH-CO-N(allyle)₂;
4. un groupe SO₂N-(CO-O-allyle)₂; ou
5. un groupe SO₂N-(CO-O-benzyle)₂;
c)
R(1) représente un groupe n-butyle;
R(2) représente un atome d'hydrogène;
R(3) représente un atome d'hydrogène;
R(15) représente
1. un groupe -SO₂-NH-CO-O-R(17) dans lequel R(17) est un groupe éthyle ou benzyle; ou
2. un groupe -SO₂-NH-CO-NH-R(17) dans lequel R(17) est un groupe n-propyle, isopropyle ou allyle;
et ses sels physiologiquement acceptables.

2. Procédé de préparation d'un composé de formule I selon la revendication 1, **caractérisé en ce que** l'on allyle des composés de formule II 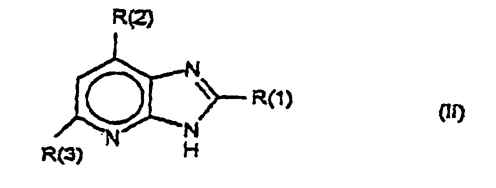 dans laquelle R(1), R(2) et R(3) ont la définition donnée ci-dessus, avec des composés de formule III 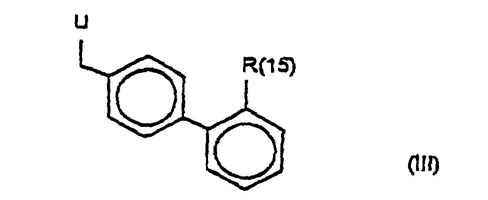 dans laquelle R(15) a la définition donnée ci-dessus et U est un groupe partant, on sépare éventuellement les groupes protecteurs introduits et on transforme éventuellement les composés de formule I obtenus en leurs sels physiologiquement acceptables.

3. Composé de formule I selon la revendication 1 à utiliser comme médicament.

4. Utilisation d'un composé de formule I selon la revendication 1 pour la préparation d'un médicament ayant une activité antagoniste des récepteurs de l'angiotensine II.

## Revendications (Revendications pour l'(les) Etat(s) contractant(s) suivant(s): ES, GR)

1. Procédé de préparation d'un composé de formule (I) 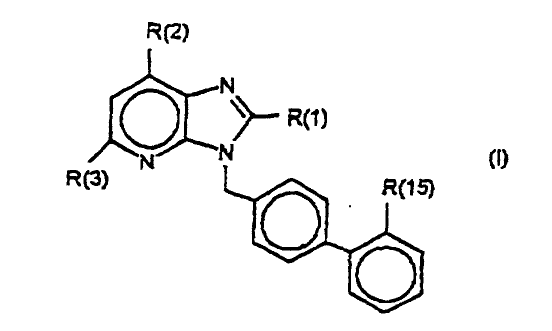 dans laquelle
a)
R(1) représente un groupe éthyle;
R(2) représente un groupe méthyle;
R(3) représente un atome d'hydrogène;
R(15) représente
1. un groupe -SO₂-NH-CO-O-R(17) dans lequel R(17) est un groupe méthyle, éthyle, propyle, vinyle ou benzyle;
2. un groupe -SO₂-NH-CO-NH-R(17) dans lequel R(17) est un groupe méthyle, éthyle, propyle, benzyle, cyclohexyle, cyclopropylméthyle ou allyle;
3. un groupe -SO₂-NH-CO-N(CH₃)-R(17) dans lequel R(17) est un groupe méthyle ou propyle; ou
4. un groupe -SO₂-NH-CO-N
b)
R(1) représente un groupe éthyle;
R(2) représente un groupe méthyle;
R(3) représente un groupe méthyle;
R(15) représente
1. un groupe -SO₂-NH-CO-O-R(17) dans lequel R(17) est un groupe méthyle, éthyle, propyle, cyclopropylméthyle, benzyle ou cyclohexylméthyle;
2. un groupe -SO₂-NH-CO-NH-R(17) dans lequel R(17) est un groupe méthyle, éthyle, propyle, allyle, cyclopropylméthyle, cyclohexylméthyle ou phényléthyle;
3. un groupe SO₂NH-CO-N(allyle)₂;
4. un groupe SO₂N-(CO-O-allyle)₂; ou
5. un groupe SO₂N-(CO-O-benzyle)₂;
c)
R(1) représente un groupe n-butyle;
R(2) représente un atome d'hydrogène;
R(3) représente un atome d'hydrogène;
R(15) représente
1. un groupe -SO₂-NH-CO-O-R(17) dans lequel R(17) est un groupe éthyle ou benzyle; ou
2. un groupe -SO₂-NH-CO-NH-R(17) dans lequel R(17) est un groupe n-propyle, isopropyle ou allyle;
et de leurs sels physiologiquement acceptables,
**caractérisé en ce que** l'on allyle des composés de formule II 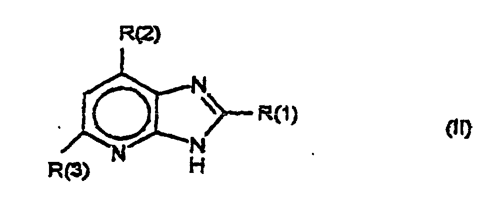 dans laquelle R(1), R(2) et R(3) ont la définition donnée ci-dessus, avec des composés de formule III 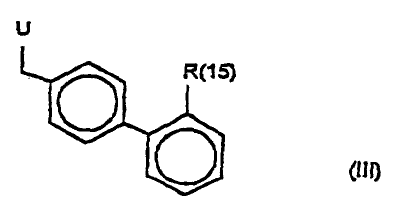 dans laquelle R(15) a la définition donnée ci-dessus et U est un groupe panant, on sépare éventuellement les groupes protecteurs introduits et on transforme éventuellement les composés de formule I obtenus en leurs sels physiologiquement acceptables.

2. Utilisation d'un composé de formule I selon la revendication 1 pour la préparation d'un médicament ayant une activité antagoniste des récepteurs de l'angiotensine II.
